# EUROPEAN PATENT SPECIFICATION

(11) **EP 1 787 652 B1**
(45) Date of publication and mention of the grant of the patent: **05.01.2011**
(21) Application number: 06123772.3
(22) Date of filing: 09.11.2006
(51) Int. Cl.: A61K 36/61, A61K 31/235, A61K 31/4174, A61P 31/10

(54) **Composition with antifungal properties, use and method**
Zusammensetzung mit antifungaler Wirksamkeit und deren Verwendung
Composition avec une propriété antifungale et son utilisation

(30) Priority: 16.11.2005 NL 1030438
(43) Date of publication of application: 23.05.2007
(73) Proprietor: Happy Foot, 5049 AD Tilburg (NL)
(72) Inventor: Vingerhoets-Jensen, Susanne Herdis Noer, 5049 AD, Tilburg (NL)
(74) Representative: Brouwer, Hendrik Rogier

(56) References cited:
- WO-A-02/087570
- WO-A2-00/15202
- WO-A2-2004/045305
- RU-C1- 2 160 585
- US-A1- 2005 271 752

## Description

The invention relates to a composition with antifungal properties. The invention also comprises a packaging comprising such a composition. The invention further comprises the use of *Melaleuca Alternifolia* essential oil in combination with at least one component chosen from the group consisting of miconazole nitrate, benzoic acid and sodium benzoate, for the preparation of such an agent. The invention moreover comprises a method for applying such a composition.

Many people suffer from fungal infections of the skin. Feet in particular are often infected with a fungus. One of the best known fungal foot infections is tinea pedis, also known as 'athlete's foot'. Tinea pedis usually occurs in the vicinity of toes, and can have a very adverse effect on the nails of the toes. Several remedies for this disorder are known which can be applied to the infected body parts, including clotrimazole, miconazole, terbinafine, ciclopirox and sulconazole. Remedies for internal use are also known, including terbinafine and itraconazole. The drawback of treatment with these known remedies is that treatments have not been found to be very successful. Fungi are not found to disappear, even after prolonged treatment, or the infection returns after a period of time. Furthermore, side-effects can occur for some of the known remedies.

Compositions comprising tea tree oi land clotrimazol for use in teh treatment of mycotic infections of hooves, paws and claws of pets and domestic animals in veterinary medicine are known from WO-A-00/ 15202.

The object of the invention is to provide an improved antifungal agent.

The invention provides for this purpose a composition with antifungal properties, comprising *Melaleuca Alternifolia* essential oil, from 0 to 99% by weight of a benzoate compound relative to the total composition, and from 0 to 99% by weight of miconazole nitrate, wherein the total percentage of the benzoate compound and miconazole nitrate together amounts to at least 0.01% by weight of the weight of the total composition. The composition according to the invention provides an improvement in the treatment of fungi on body parts, in particular foot fungi. In addition, the composition is also particularly effective as preventive antifungal remedy. *Melaleuca Alternifolia* essential oil is found to enhance the per se known effect of both miconazole nitrate and benzoate compounds.

*Melaleuca Alternifolia* essential oil is an etheric oil obtained from the tea tree, also known as Manuka. Although types of tea tree other than *Melaleuca Alternifolia* are known, *Melaleuca Alternifolia* is the most readily available. The essential oil is, in contrast to many other etheric oils, not irritating to the skin. Typical chemical compounds found in *Melaleuca Alternifolia* essential oil are terpinene-4-ol and cineole.

The best known benzoate compounds are benzoic acid and sodium benzoate, although other salts of benzoic acid can also be used. The stated percentage by weight assumes sodium benzoate, though it will be apparent that when other benzoate salts are used the percentages must be modified to the relevant molecular weight. A choice is preferably made for a benzoate with a good solubility in a determined composition, wherein sodium benzoate is particularly suitable for polar compositions (for instance an aqueous composition), while benzoic acid is particularly suitable for apolar compositions (for instance an oleaginous composition).

Miconazole nitrate is a known antifungal compound. Other salts of miconazole can also be used, as well as active derivatives of miconazole.

The composition preferably comprises at least 2% by weight of *Melaleuca Alternifolia* essential oil and from 0.01 to 99% by weight of a benzoate compound. Compositions comprising active quantities of benzoate compound in combination with *Melaleuca Alternifolia* essential oil thus provide a better antifungal effect than comparable compositions with benzoate compound without *Melaleuca Alternifolia* essential oil.

In another preferred embodiment the composition comprises at least 2% by weight of *Melaleuca Alternifolia* essential oil and from 0.01 to 99% by weight of miconazole nitrate. Compositions comprising active quantities of miconazole nitrate in combination with *Meloleuca Alternifolia* essential oil provide a better antifungal effect than comparable compositions with miconazole nitrate without *Melaleuca Alternifolia* essential oil.

The composition most preferably comprises at least 2% by weight of *Melaleuca Alternifolia* essential oil, from 0.01 to 99% by weight of a benzoate compound and from 0.01 to 99% by weight of miconazole nitrate. The combination of *Melaleuca Alternifolia* essential oil, a benzoate compound and miconazole nitrate is even found to provide a still greater synergistic antifungal effect.

In a preferred embodiment the composition comprises at least between 2 and 20% by weight of *Melaleuca Alternifolia* essential oil. Although higher concentrations give a better antifungal effect, an optimum between activity and economic use of *Melaleuca Alternifolia* essential oil is achieved at this concentration. The remainder of the weight of the composition can for instance be filled with a relatively inexpensive diluent, which can be either solid or liquid, or a mixture thereof.

It is advantageous if the composition comprises between 2 and 8% by weight of *Melaleuca Alternifolia* essential oil and between 0.5 and 1% by weight of a benzoate compound relative to the total composition. At this concentration an optimum is achieved between antifungal activity and economic use of *Melaleuca Alternifolia* essential oil and benzoate compound.

In another preferred embodiment the composition comprises between 2 and 8% by weight of *Melaleuca Alternifolia* essential oil and between 0.1 and 2% by weight of miconazole nitrate. At this concentration an optimum is achieved between antifungal activity and economic use of *Melaleuca Alternifolia* essential oil and miconazole nitrate.

In yet another preferred embodiment the composition comprises between 2 and 8% by weight of *Melaleuca Alternifolia* essential oil, between 0.5 and 1% by weight of a benzoate compound and between 0.1 and 2% by weight of miconazole nitrate. At this concentration an optimum is achieved between antifungal activity and economic use of *Melaleuca Alternifolia* essential oil, the benzoate compound and miconazole nitrate.

It is advantageous if the composition also comprises a dermatologically acceptable diluent. A diluent dilutes the active components (*Melaleuca Alternifolia* essential oil in combination with the benzoate compound and/or miconazole nitrate), so that an optimal distribution is achieved during application to a surface for treating. Ideally the active components are mixed substantially homogeneously with the rest of the composition. Different solid, liquid and/or gaseous substances or compositions can be used as diluents. The diluent can for instance be a solvent in which the active components dissolve. Liquid diluents which can be used are for instance water, aliphatic alcohols and other organic solvents. The composition can also be an emulsion, such as a lotion, ointment or cream. In a particular preferred embodiment the diluent is a gas, wherein the composition is formed into a foam. Another option is a solid substance, wherein the resulting composition can for instance be processed into a powder. In order to obtain the above stated compositions use generally has to be made of suitable excipients known in the cosmetic and pharmaceutical industry, such as surfactants, fillers and emulsifiers, which enhance mixing of the active components with the diluents and bring about the desired structure of the composition.

It is advantageous if the diluent is substantially a volatile solvent. A volatile solvent makes it possible to apply the active components in diluted distribution over a large surface, wherein after evaporation of the volatile solvent the active components remain behind in more concentrated form. A considerable antifungal effect is thus achieved. Volatile solvents are understood to mean liquids with a lower heat of evaporation than water (2.26 x 10⁶ J/kg). Examples are acetone, methyl ethyl ketone, water, ethanol and mixtures thereof. The volatile solvent preferably comprises at least 50% by weight of the composition.

In a particular preferred embodiment the diluent is substantially ethanol. Ethanol has the particular advantage that when it evaporates it also extracts the water from the treated surface, thereby improving the antifungal effect. Another advantage of ethanol is that in concentrations greater than 10% by weight it also acts as preservative for the composition. In addition, ethanol has a good capacity for dissolving the active components, whereby a homogeneous composition can be more readily obtained.

In a preferred embodiment the composition also comprises an aromatic substance. *Melaleuca Alternifolia* essential oil has an odour which is perceived by some as unpleasant, and this can be masked by an active quantity of aromatic substance. Diverse aromatic substances which can be used are commercially available, such as cinnamon, eucalyptus and menthol. Particularly suitable for this purpose are fruit aromas which have the advantage that only a relatively small quantity hereof is necessary to mask the odour of tea tree oil. Examples of suitable aromas are lemon and lime.

The invention also comprises a packaging comprising a composition according to the invention. For liquid compositions it is possible to envisage a bottle or other reclosable packaging. The packaging is preferably aroma-tight. The packaging more preferably comprises a predetermined quantity for treatment of a surface. The quantity can for instance be apportioned for the treatment of two feet. A separate package is thus used for each treatment, which improves hygiene. A plastic disposable package can be envisaged here, for instance a closed breakable bag having therein a powder or application fluid.

It is advantageous if the packaging also comprises an applicator adapted to apply the composition to a surface. An applicator can be envisaged here which is connected to a container for the composition, for instance a spray head for distributing a liquid composition according to the invention over a surface. A brush or sponge for applying the liquid can for instance also be envisaged. For the treatment of larger surfaces, for instance the floor of a sports hall or swimming pool, larger applicators can be envisaged such as a floor cloth for liquid compositions according to the invention.

The invention further relates to the use of *Melaleuca Alternifolia* essential oil in combination with at least one component chosen from the group consisting of miconazole nitrate, benzoic acid and sodium benzoate for the preparation of an antifungal remedy. The combinations of known antifungal substances with *Melaleuca Alternifolia* essential oil according to the invention provide an improved antifungal effect.

The invention moreover comprises a method comprising of applying a composition according to the invention to a surface. Such a surface is thus protected in preventive manner from fungal infections. Existing fungal infections are also combatted by applying the composition according to the invention to a surface on a regular basis (preferably daily). The method can for instance be applied to surfaces in bathrooms, swimming pools and sports accommodation. The surface can also be a skin surface, in particular a foot, more particularly a toenail. When used on toenails, it is recommended to first remove as much as possible of the infected parts of the toenail before applying the composition. This results in a shorter period of treatment.

The invention will now be elucidated on the basis of the following examples.

The compositions A, B, C, D, E, F according to the invention shown in table I were prepared by mixing the stated components by means of known methods. Formulations A-F are liquid formulations, although formulations in powder form can also be envisaged.

Formulation A is a formulation of tea tree oil (*Melaleuca Alternifolia* essential oil) with sodium benzoate.

Formulation B is a formulation of *Melaleuca Alternifolia* essential oil with miconazole nitrate.

Formulation C comprises *Melaleuca Alternifolia* essential oil in combination with sodium benzoate and miconazole nitrate.

Formulation D is a solution of benzoic acid in *Melaleuca Alternifolia* essential oil.

Formulation E is an evaporable solution of *Melaleuca Alternifolia* essential oil in miconazole nitrate in ethanol.

Formulation F is an evaporable solution of *Melaleuca Alternifolia* essential oil with miconazole nitrate in ethanol.

**Table I: Antifungal formulations**

| components | percent by weight | | | | | |
|---|---|---|---|---|---|---|
| Formulation | A | B | C | D | E | F |
| glycerol | - | 20 | - | - | 20 | 20 |
| PEG-40 Hydrogenated Castor oil | 2 | - | 2 | - | - | - |
| PEG-7 Glyceryl Cocoate | 20 | - | 20 | - | - | - |
| Tea Tree oil | 4 | 4 | 4 | 95 | 15 | 15 |
| miconazole nitrate | - | 0.17 | 0.17 | - | 0.5 | 0.5 |
| Sodium benzoate | 0.59 | - | 0.59 | - | - | 0.59 |
| Benzoic acid | - | - | - | 1 | - | - |
| Olive oil | 1 | - | - | 4 | - | - |
| Ethanol | 20 | 20 | 20 | - | rest | rest |
| Water | rest | rest | rest | - | - | - |

Excipients are also used in addition to the active components (*Melaleuca Alternifolia* essential oil, benzoate and miconazole nitrate). These excipients can if desired be replaced by other known substances with similar properties. Glycerol assists dissolving and homogenizing. PEG-40 Hydrogenated Castor oil is an emulsifier and surfactant which assists good mixing of the active components. PEG-7 Glyceryl Cocoate is a surfactant which also softens the skin. Olive oil also softens the skin.

The different formulations were applied in a comparative test. A first test consisted of qualitative determination of the preventive antifungal effect. It was determined here to what extent the formulation was able to prevent fungal growth on a previously clean surface. The therapeutic effect of the compositions was further tested. Diverse people with foot fungus were herein treated daily with the composition for a period of six months. This is the period of time a nail needs on average to renew itself through growth. A usable antifungal agent stops the growth of the fungus such that the fungus no longer occurs in the renewed nail after six months. Table II shows the results of the different types of composition for both preventive and therapeutic effect. The assessments are represented qualitatively, with the following meaning: (-: unsatisfactory/no antifungal effect, + satisfactory antifungal effect, ++ good antifungal effect, +++ very good antifungal effect). The differences between satisfactory, good and very good antifungal effect are determined by the quantity of formulation necessary to achieve the desired result, wherein less of the very good formulations is required to arrive at a comparable result.

**Table II: Preventive and therapeutic antifungal effect**

| Ingredient | Preventive Antifungal effect | Therapeutic Antifungal effect |
|---|---|---|
| Tea tree oil | - | - |
| sodium benzoate | + | + |
| miconazole nitrate | + | + |
| Tea tree oil + benzoate (A) | ++ | ++ |
| Tea tree oil + miconazole nitrate (B) | ++ | ++ |
| Tea tree oil + benzoate + miconazole nitrate (C) | +++ | +++ |

The above examples are non-limitative, and many variants of the composition according to the invention can be envisaged by a skilled person in the field. In particular, the ingredients which are not immediately significant for the antifungal effect can be replaced by other constituents known in dermatology.

## Claims

1. Composition for use in the treatment of fungi on a foot, comprising *Melaleuca Alternifolia* essential oil,
from 0 to 99% by weight of a benzoate compound relative to the total composition, and
from 0 to 99% by weight of miconazole nitrate,
**characterized in that**
the total percentage of the benzoate compound and miconazole nitrate together amounts to at least 0.01% by weight of the weight of the total composition, and
the composition comprises at least between 2 and 20% by weight of *Melaleuca Alternifolia* essential oil.

2. Composition as claimed in claim 1, **characterized in that**
the composition comprises at least 2% by weight of *Melaleuca Alternifolia* essential oil and from 0.01 to 99% by weight of a benzoate compound.

3. Composition as claimed in claim 1, **characterized in that**
the composition comprises at least 2% by weight of *Melaleuca Alternifolia* essential oil and from 0.01 to 99% by weight of miconazole nitrate.

4. Composition as claimed in claim 1, **characterized in that**
the composition comprises at least 2% by weight of *Melaleuca Alternifolia* essential oil,
from 0.01 to 99% by weight of a benzoate compound, and
from 0.01 to 99% by weight of miconazole nitrate.

5. Composition as claimed in any of the foregoing claims, **characterized in that** the composition comprises between 2 and 8% by weight of *Melaleuca Alternifolia* essential oil and between 0.5 and 1% by weight of a benzoate compound relative to the total composition.

6. Composition as claimed in any of the foregoing claims, **characterized in that** the composition comprises between 2 and 8% by weight of *Melaleuca Alternifolia* essential oil and between 0.1 and 2% by weight of miconazole nitrate.

7. Composition as claimed in any of the foregoing claims, **characterized in that** the composition comprises between 2 and 8% by weight of *Melaleuca Alternifolia* essential oil, between 0.5 and 1% by weight of a benzoate compound and between 0.1 and 2% by weight of miconazole nitrate.

8. Composition as claimed in any of the foregoing claims, **characterized in that** the composition also comprises a dermatologically acceptable diluent.

9. Composition as claimed in claim 8, **characterized in that** the diluent is substantially a volatile solvent.

10. Composition as claimed in claim 10, **characterized in that** the diluent is substantially ethanol.

11. Composition as claimed in any of the foregoing claims, **characterized in that** the composition also comprises an aromatic substance.

12. Packaging for use in the treatment of fungi on a foot comprising a composition as claimed in any of the foregoing claims 1-11.

13. Packaging as claimed in claim 12, **characterized in that** the packaging also comprises an applicator adapted to apply the composition to a surface.

14. Use of *Melaleuca Alternifolia* essential oil in combination with at least one component chosen from the group consisting of miconazole nitrate, benzoic acid and sodium benzoate for the preparation of an antifungal agent.

## Patentansprüche

1. Zusammensetzung zur Anwendung in der Pilzbehandlung an einem Fuß, die ätherisches Öl vom Teebaum *Melaleuca Alternifolia* umfasst, sowie von 0 bis 99 Gew.-% einer Benzoatverbindung bezogen auf die Gesamtzusammensetzung und
von 0 bis 99 Gew.-% Miconazolnitrat,
**dadurch gekennzeichnet, dass**
der Gesamtanteil der Benzoatverbindung und des Miconazolnitrats zusammen mindestens 0,01 Gew.-% des Gewichts der Gesamtverbindung beträgt, und
die Zusammensetzung mindestens zwischen 2 und 20 Gew.-% *Melaleuca Alternifolia* ätherisches Öl umfasst.

2. Zusammensetzung nach Anspruch 1, **dadurch gekennzeichnet, dass**
die Zusammensetzung mindestens 2 Gew.-% *Melaleuca Alternifolia* ätherisches Öl und von 0,01 bis 99 Gew.-% einer Benzoatverbindung umfasst.

3. Zusammensetzung nach Anspruch 1, **dadurch gekennzeichnet, dass**
die Zusammensetzung mindestens 2 Gew.-% *Melaleuca Alternifolia* ätherisches Öl und von 0,01 bis 99 Gew.-% Miconazolnitrat umfasst.

4. Zusammensetzung nach Anspruch 1, **dadurch gekennzeichnet, dass**
die Zusammensetzung mindestens 2 Gew.-% *Melaleuca Alternifolia* ätherisches Öl sowie
von 0,01 bis 99 Gew.-% einer Benzoatverbindung und
von 0,01 bis 99 Gew.-% Miconazolnitrat umfasst.

5. Zusammensetzung nach einem der vorhergehenden Ansprüche, **dadurch gekennzeichnet, dass** die Zusammensetzung zwischen 2 und 8 Gew.-% *Melaleuca Alternifolia* ätherisches Öl und zwischen 0,5 und 1 Gew.-% einer Benzoatverbindung bezogen auf die Gesamtzusammensetzung umfasst.

6. Zusammensetzung nach einem der vorhergehenden Ansprüche, **dadurch gekennzeichnet, dass** die Zusammensetzung zwischen 2 und 8 Gew.-% *Melaleuca Alternifolia* ätherisches Öl und zwischen 0,1 und 2 Gew.-% Miconazolnitrat umfasst.

7. Zusammensetzung nach einem der vorhergehenden Ansprüche, **dadurch gekennzeichnet, dass** die Zusammensetzung zwischen 2 und 8 Gew.-% *Melaleuca Alternifolia* ätherisches Öl, zwischen 0,5 und 1 Gew.-% einer Benzoatverbindung und zwischen 0,1 und 2 Gew.-% Miconazolnitrat umfasst.

8. Zusammensetzung nach einem der vorhergehenden Ansprüche, **dadurch gekennzeichnet, dass** die Zusammensetzung auch ein dermatologisch verträgliches Verdünnungsmittel umfasst.

9. Zusammensetzung nach Anspruch 8, **dadurch gekennzeichnet, dass** das Verdünnungsmittel ein im Wesentlichen flüchtiges Lösungsmittel ist.

10. Zusammensetzung nach Anspruch 10, **dadurch gekennzeichnet, dass** das Lösungsmittel im Wesentlichen Ethanol ist.

11. Zusammensetzung nach einem der vorhergehenden Ansprüche, **dadurch gekennzeichnet, dass** die Zusammensetzung auch einen Aromastoff umfasst.

12. Packung zur Anwendung in der Pilzbehandlung an einem Fuß, die eine Zusammensetzung nach einem der vorhergehenden Ansprüche 1-11 umfasst.

13. Packung nach Anspruch 12, **dadurch gekennzeichnet, dass** die Packung auch einen Applikator umfasst, der geeignet ist, die Zusammensetzung auf einer Oberfläche zu applizieren.

14. Die Anwendung von *Melaleuca Alternifolia* ätherischem Öl in Verbindung mit mindestens einer Verbindung aus der Gruppe bestehend aus Miconazolnitrat, Benzoatverbindung und Natriumbenzoat für die Bereitung eines Antipilzmittels.

## Revendications

1. Composition à utiliser dans le traitement des mycoses du pied, comprenant :
de l'huile essentielle de *Melaleuca alternifolia;*
de 0 à 99 % en poids d'un composé de benzoate par rapport à la composition totale, et
de 0 à 99 % en poids de nitrate de miconazole,
**caractérisée en ce que** :
le pourcentage total du composé de benzoate et du nitrate de miconazole conjointement se monte à au moins 0,01 % en poids du poids de la composition totale, et **en ce que** la composition comprend au moins entre 2 et 20 % en poids d'huile essentielle de *Melaleuca alternifolia.*

2. Composition selon la revendication 1, **caractérisée en ce que** :
la composition comprend au moins 2 % en poids d'huile essentielle de *Melaleuca alternifolia* et de 0,01 à 99 % en poids d'un composé de benzoate.

3. Composition selon la revendication 1, **caractérisée en ce que** :
la composition comprend au moins 2 % en poids d'huile essentielle de *Melaleuca alternifolia* et de 0,01 à 99 % en poids de nitrate de miconazole.

4. Composition selon la revendication 1, **caractérisée en ce que** :
la composition comprend au moins 2 % en poids d'huile essentielle de *Melaleuca alternifolia,*
de 0,01 à 99 % en poids d'un composé de benzoate, et
de 0,01 à 99 % en poids de nitrate de miconazole.

5. Composition selon l'une quelconque des revendications précédentes, **caractérisée en ce que** la composition comprend entre 2 et 8 % en poids d'huile essentielle de *Melaleuca alternifolia* et entre 0,5 et 1 % en poids d'un composé de benzoate par rapport à la composition totale.

6. Composition selon l'une quelconque des revendications précédentes, **caractérisée en ce que** la composition comprend entre 2 et 8 % en poids d'huile essentielle de *Melaleuca alternifolia* et entre 0,1 et 2 % en poids de nitrate de miconazole.

7. Composition selon l'une quelconque des revendications précédentes, **caractérisée en ce que** la composition comprend entre 2 et 8 % en poids d'huile essentielle de *Melaleuca alternifolia,* entre 0,5 et 1 % en poids d'un composé de benzoate et
entre 0,1 et 2 % en poids de nitrate de miconazole.

8. Composition selon l'une quelconque des revendications précédentes, **caractérisée en ce que** la composition comprend aussi un diluant acceptable sur le plan dermatologique.

9. Composition selon la revendication 8, **caractérisée en ce que** le diluant est sensiblement un solvant volatil.

10. Composition selon la revendication 10, **caractérisée en ce que** le diluant est sensiblement de l'éthanol. 1.

11. Composition selon l'une quelconque des revendications précédentes, **caractérisée en ce que** la composition comprend aussi une substance aromatique.

12. Conditionnement à utiliser dans le traitement des mycoses du pied comprenant une composition selon l'une quelconque des revendications 1-11.

13. Conditionnement selon la revendication 12, **caractérisée en ce que** le conditionnement comprend aussi un applicateur adapté pour appliquer la composition sur une surface.

14. Utilisation d'huile essentielle de *Melaleuca alternifolia* en association avec au moins un composant choisi dans le groupe constitué du nitrate de miconazole, de l'acide benzoïque et du benzoate de sodium pour préparer un agent antifongique.
